(19) Europäisches Patentamt
European Patent Office
Office européen des brevets

(11) Publication number: **0 074 856**
**A2**

(12) **EUROPEAN PATENT APPLICATION**

(21) Application number: 82304864.0

(22) Date of filing: 15.09.82

(51) Int. Cl.[3]: C 07 C 49/627
C 07 C 49/693, C 07 C 45/78
C 07 B 19/00, C 07 C 87/28

(30) Priority: 16.09.81 GB 8127981

(43) Date of publication of application: 23.03.83 Bulletin 83/12

(84) Designated Contracting States: AT BE CH DE FR GB IT LI NL SE

(71) Applicant: GLAXO GROUP LIMITED
Clarges House 6-12 Clarges Street
London W1Y 8DH(GB)

(72) Inventor: Wallis, Christopher J.
The White House Noons Folly Newmarket Road
Royston Hertfordshire(GB)

(74) Representative: Skailes, Humphrey John et al,
Frank B. Dehn & Co. Imperial House 15-19 Kingsway
London WC2B 6UZ(GB)

(54) **Resolution of racemic bicycloheptenones.**

(57) A method is described of resolving a racemic bicycloheptenone of formula (1)

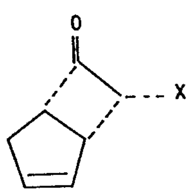

(1)

(in which X is a hydrogen, chlorine or bromine atom) which comprises forming an α-hydroxysulphonic acid - chiral amine salt thereof of formula (2)

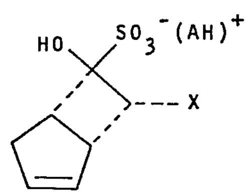

(2)

(in which A is the chiral amine), separating the diastereomeric salts and regenerating the bicycloheptenone of formula (1) in optically active form from the separated salt.

The resolved bicycloheptenones are useful in the stereospecific synthesis of prostanoids and other substituted cyclopentanones.

EP 0 074 856 A2

RESOLUTION OF RACEMIC BICYCLOHEPTENONES

Bicyclo [3.2.0] hept-2-en-6-one and its precursors are useful intermediates in the synthesis of prostanoids and other substituted cyclopentanes. The bicycloheptenone has previously been used in its racemic form in such syntheses, and where a specific enantiomeric form of the final product has been required resolution has been effected at a later stage in the reaction sequence.

We have now found that the amine bisulphite addition compounds of the bicycloheptenone and certain of its halo derivatives can be readily separated, and that this method can be used as the basis of an economically attractive stereospecific synthesis of naturally occurring prostanoids and other substituted cyclopentanes.

The invention thus provides a method of resolving a racemic bicycloheptenone of formula (1)

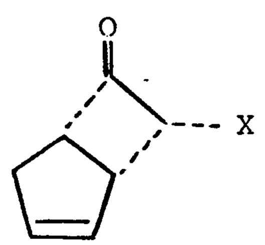

(1)

(in which X is a hydrogen, chlorine or bromine atom) which comprises forming an α-hydroxysulphonic acid - chiral amine salt thereof of formula (2)

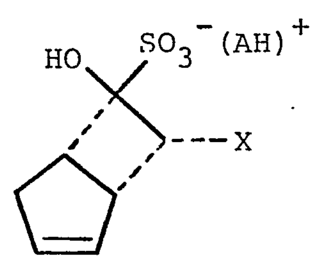

(2)

(in which A is the chiral amine), separating the diastereomeric salts and regenerating the bicycloheptenone of formula (1) in optically active form from the separated salt.

The salts of formula (2) are new compounds. Compounds of formula (I) in which X is a chlorine or bromine atom have not previously been resolved and thus their separated enantiomers are also new compounds.

Chiral amines which may be used include S-(-)-1-phenylethylamine, R-(+)-1-phenylethylamine, (+)-ephedrine, (-)-ephedrine, R-(-)-2-amino-1-butanol, 1S,2S-(+)-2-amino-1-phenyl-1,3-propane diol, (-)-quinine, (+)-quinidine, (-)-brucine, (+)-dehydroabietylamine, R-(+)-1-naphthyl-1-ethylamine, and S-(-)-1-naphthyl-1-ethylamine. The R-(+)-1-phenylethylamine salt is preferred.

The α-hydroxysulphonic acid-amine salt may be formed in any convenient way. For example, the bicycloheptenone may be mixed in solution with an amine-bisulphite salt, prepared for example by first treating the chiral amine (e.g. at room temperature) with a solution of a bisulphite (e.g. sodium metabisulphite) and then adjusting the pH to 6-7 (e.g. with acetic acid).

Alternatively, the bicycloheptenone may be treated with an amine-bisulphite complex obtained by passing sulphur dioxide through an approximately equimolar solution of the chiral amine and water in an organic solvent. The solvent may for example be ether, dichloromethane or acetonitrile and the reaction may be effected at room temperature. The amine-bisulphite salt, $(AH)^+(HSO_3)^-$, which is initially formed is not isolated, and further sulphur dioxide is introduced until a homogeneous liquid complex is obtained. The ketone is conveniently dissolved in a suitable solvent, such as dichloromethane, before treatment with the complex.

The diastereomeric mixture of the salts of formula (2) may be separated by known methods. Fractional crystallisation from an inert solvent is preferably used, suitable solvents being water-miscible organic solvents, such as ethanol or isopropanol, or dichloromethane. Separation may also be effected by chromatography, e.g. on silica using an organic solvent.

Regeneration of the optically active bicycloheptenone may be effected by treatment of the resolved salt with either a base (e.g. sodium carbonate) or an acid (e.g. ethanolic HCl), at any convenient temperature (e.g. room temperature to 60°C).

The resolved bicyclo [3.2.0]hept-2-en-6-one obtained by this method may be used in the synthesis of naturally occurring prostanoids and other substituted cyclopentanes, using for example the methods described in British Patent Specifications 1568371, 2028805A, 2070591A and 2075503A and in the example below.

The method of the invention is particularly important in the synthesis of [IR-[1α(Z),2β,5α]]-(-)-7-[5-[[(1,1'-biphenyl)-4-yl]methoxy]-2-(4-morpholinyl )-3-oxocyclopentyl]-4-heptenoic acid, for example by the method described in the example below.

Resolved compounds of formula (1) in which X is a chlorine or bromine atom should be converted to bicyclo [3.2.0]hept-2-en-6-one before use in these methods. The dehalogenation may for example be effected by heating in the presence of zinc and glacial acetic acid.

The following examples illustrate the invention "Jones reagent" is a solution of chromic acid and sulphuric acid in water. A 2.67M solution contains $CrO_3$ (26.7g) and concentrated $H_2SO_4$ (23ml) made up to 100ml with water.

Temperatures are in °C. The following abbreviations are used:

TLC - thin layer chromatography using $SiO_2$; PE - petroleum ether, boiling at 40-60°; DIBAL - diisobutylaluminium hydride; THF - tetrahydrofuran; ER - ether; EA - ethyl acetate.

Chromatography was carried out using silica gel unless otherwise stated. Drying was effected with $MgSO_4$ unless otherwise stated. "Hyflo" is a filtration aid.

EXAMPLE 1

(-)-6-Hydroxybicyclo[3.2.0]hept-2-en-6-sulphonic acid, compound with (+)-α-methylbenzenemethanamine (1:1)

Sulphur dioxide was bubbled (ca. 1500 cc/min) into a stirred

solution of (+)-α-methylbenzenemethanamine (24.2g) and water (4 ml) in $CH_2Cl_2$ (200 ml) for 30 min. A solution of (±)-bicyclo[3.2.0]hept-2-en-6-one (21.6g) in $CH_2Cl_2$ (20 ml) was then added and the mixture stirred at 20° until crystallisation started. The mixture was then "aged" at 4° for 18h before filtration. The solid product was then washed ($CH_2Cl_2$, 2 x 40 ml) and dried under reduced pressure (35.7g). $[\alpha]_D^{23} = -8.2°$ (c = 5, water).

The crude product (33g) was then dissolved in refluxing propan-2-ol (99 ml), filtered, and stirred with seeding to induce crystallisation. The mixture was aged at 4° for 18h, filtered and the solid washed with propan-2-ol (2 x 10 ml) before drying under reduced pressure to give the <u>title compound</u> (21.9g), m.p. 86 - 88°. $[\alpha]_D^{23} = -14.8°$ (c = 5, water)

<u>EXAMPLE 2</u>

<u>(-)-Bicyclo[3.2.0]hept-2-en-6-one</u>

2N Aqueous $Na_2CO_3$ (84 ml) was added to the resolved amine bisulphite salt (14g) prepared in Example 1 and the mixture stirred until a solution was formed (ca. 5 min.) The mixture was then extracted with $CH_2Cl_2$ (1 x 30 ml, 3 x 20 ml) and the combined extracts washed with 2N HCl (28 ml) and dried ($Na_2SO_4/K_2CO_3$). After evaporation under reduced pressure the residue was distilled (b.p. 100°/15 mm Hg) to yield the <u>title compound</u> as a colourless oil (3.8g), $[\alpha]_D^{23}$ = -33.2° (c = 5, MeOH).

EXAMPLE 3

This example describes the preparation of [1R-[1α(Z),2β,5α]]-(-)-7-[5-[[(1,1'-biphenyl)-4-yl]methoxy]-2-(4-morpholinyl)-3-oxocyclopentyl]-4-heptenoic acid from (-)-bicyclo[3.2.0]hept-2-en-6-one prepared by the method of the invention.

Intermediate 1

[1R-(exo,endo)]-(-)-2-Bromo-3-hydroxybicyclo[3.2.0]heptan-6-one

1,3-Dibromo-5,5-dimethyl hydantoin (1.33g) was added to a solution of (-)-bicyclo-[3.2.0]hept-2-en-6-one (1g) in acetone (16.5 ml) and water (4.25 ml). The mixture was then stirred for 18h at 20° before water (8 ml) was added and the remaining oxidising agent destroyed by the dropwise addition of aqueous sodium bisulphite. The acetone was evaporated under reduced pressure and the aqueous residue extracted with $CH_2Cl_2$ (3 x 20 ml). The combined extracts were dried, filtered and evaporated to give a crude sample of the title compound as a solid (1.82 g, $[\alpha]_D^{22}$= -55°). A sample (1 g) was purified by chromatography using 1:4 EA-PE (b.p. 60 - 80°) as eluent to give material (0.74 g) of m.p. 84 - 86° $[\alpha]_D^{22}$= -57.3°.

Intermediate 2

[1R-(endo,anti)]-(+)-5-Hydroxy-7-(4-morpholinyl)bicyclo-[2.2.1]heptan-2-one

A solution of [1R-(exo,endo )]-(-)-2-bromo-3-hydroxybicyclo[3.2.0]heptan-6-one (8.82g) in $CH_2Cl_2$ (85ml) containing morpholine (15ml) was stirred at room temperature for 20h. The precipitate was filtered off and washed with $CH_2Cl_2$ (100ml). The combined filtrates were washed with $NaHCO_3$ solution and water (75ml each) dried and evaporated to give a semi-solid which was chromatographed on silica using EA as eluent.

The title compound was obtained as a solid which crystallised from 1:1 EA-PE (b.p. 60-80°) to give material (6.1g) of m.p. 137-139°. $[\alpha]_D^{20}$=+55.73° (MeOH).

Intermediate 3

[1R-(endo,anti)]-(+)-5-[[(1,1'-Biphenyl)-4-yl]methoxy]-7-(4-morpholinyl)bicyclo[2.2.1]heptan-2-one

A mixture of Intermediate 2 (10.45g), benzyl triethylammonium chloride (1.5g) and biphenylmethyl bromide (15.3g) in $CH_2Cl_2$ (50ml) was cooled to 0° whilst NaOH (12g) in water (20ml) was added. The two phases were stirred vigorously for 24h at 20°. The mixture was diluted with water (120ml) and extracted with $CH_2Cl_2$ (3 x 100ml). The combined extracts were washed with brine (2 x 50ml), dried and evaporated, and the residue triturated with ER (100ml) to give a solid (16g). The solid was crystallised from iso-propyl acetate (120ml) to give the title compound (9.6g) as platelets, m.p. 138-140°. $[\alpha]_D^{21}$=+22.12° ($CHCl_3$).

Intermediate 4

[1R-(endo,anti)]-(-)-6-[[(1,1'-Biphenyl)-4-yl]methoxy]-8-(4-morpholinyl-2-oxabicyclo[3.2.1]octan-3-one

Peracetic acid (8.7ml, 6.12 M) was added dropwise to a stirred solution of Intermediate 3 (5g) in $CH_2Cl_2$ (25ml) at 0°. The mixture was stirred for 24h while allowing the temperature to rise to ambient. 20% w/w $Na_2SO_3$ in water (60ml) was added dropwise at 0° and the mixture was stirred at room temperature for 0.75h. Iso-propyl acetate (25ml) was added and the layers were separated. The aqueous layer was extracted with (1:1) isopropyl acetate-$CH_2Cl_2$ (2 x 25ml), and the combined organic layers were

washed with 1N NaOH (2 x 50ml) and brine (50ml) then dried and evaporated to give a solid (3.3g). The solid was crystallised from 1:1 EA-PE (80ml) to give the title compound as prisms (6.9g), m.p. 147-148°. $[\alpha]_D^{21.5}=-26.44°$ ($CHCl_3$).

Intermediate 5

[1R-(1α,2β,3α,5α)]-5-[[(1,1'-Biphenyl)-4-yl]methoxy]-3-hydroxy-2-(4-morpholinyl)cyclopentane acetaldehyde

A solution of Intermediate 4 (3g) in dry $CH_2Cl_2$ (60ml) was cooled (-78°) and stirred under nitrogen whilst a solution of DIBAL in hexane (10.7ml, 1.43 M) was added dropwise. Methanol (60ml) was added dropwise at -78° and the cooling bath was removed. After stirring at room temperature for 2h the precipitate was filtered off and was washed well with methanol. The combined filtrates were evaporated in vacuo and the residue was dissolved in $CH_2Cl_2$ (100ml), dried, filtered and evaporated to give the title compound as a foam (2.95g). IR ($CHBr_3$) 3580, 1718 $cm^{-1}$.

Intermediate 6

[1R-(1α,2β,3α,5α)]-5-[[1,1'-Biphenyl)-4-yl]methoxy]-3-hydroxy-2-(4-morpholinyl)cyclopentanepropanal

(Methoxymethyl)triphenylphosphonium chloride (7.15g) was added to a stirred solution of potassium tert.-butoxide (2.35g) in dry THF (40ml) under nitrogen. After 15 min a solution of Intermediate 5 (2.75g) in dry THF (20ml) was added dropwise and stirring continued for 30 min.

The reaction mixture was poured into 2N hydrochloric acid (50ml) at 0° and was stirred at 10-15° for 1.5h. The mixture was adjusted to about pH 10 with saturated $K_2CO_3$ solution and extracted with $CH_2Cl_2$ (3 x 100ml). The combined extracts were washed with brine (100ml), dried and evaporated and the residue chromatographed on silica using 9:1 EA-methanol as eluent to give the title compound as a foam (2.47g). TLC 9:1 EA-methanol Rf 0.3.

Intermediate 7

[1R-[1α(Z),2β,3α,5α]]-(+)-7-[5-[[(1,1'-Biphenyl)-4-yl]methoxy]-3-hydroxy-2-(4-morpholinyl)cyclopentyl]-4-heptenoic acid, hydrochloride

Dry THF (90ml) was added to a stirred mixture of potassium *tert*.-butoxide (2.46g) and 3-(carboxypropyl)-triphenylphosphonium bromide (4.6g) under nitrogen. After about 30 min Intermediate 6 (2.25g) in dry THF (50ml) was added dropwise and stirring continued for 2.5h. Water (25ml) was added and most of the THF was removed *in vacuo*. The residue in water (50ml) and 2N NaOH (20ml) was extracted with EA (2 x 50ml). The aqueous layer was adjusted to pH 6 with buffer (1M $KH_2PO_4$ 3 parts, 1M $Na_2HPO_4$ 1 part) and was extracted with $CH_2Cl_2$ (3 x 50ml). The combined extracts were washed with brine, dried and evaporated to give a foam (2.7g). This material was dissolved in EA (100ml) and treated with an excess of ethereal hydrogen chloride. After cooling at 0° for 16h the salt was collected and washed with 1:1 ER-EA (25ml) followed by ER (40ml). Crystallisation from 5:1 EA-methanol gave the title compound (1.6g) as prisms, m.p. 152-153°. $[\alpha]_D^{21}=+54°$ ($CHCl_3$).

Example 3

[1R-[1α(Z),2β,5α]]-(-)-7-[5-[[(1,1'-Biphenyl)-4-yl]methoxy]-2-(4-morpholinyl)-3-oxocyclopentyl]-4-heptenoic acid

Jones reagent (1.83ml, 2.67M) was added to a stirred mixture of 'hyflo' (4.8g) and the free base of Intermediate 7 (1.2g) in acetone (40ml) at 5° and stirring was continued for 40 min. Isopropanol (1.8ml) was added dropwise and after 10 min the hyflo was removed by filtration and was washed with acetone (30ml) and pH 5 buffer (50ml, $KH_2PO_4$ and $Na_2HPO_4$ in water). The combined filtrates were

evaporated *in vacuo* at 10-15° to remove most of the acetone. The residue was diluted with pH 5 buffer (25ml) and extracted with ER (4x50ml). The combined extracts were washed with pH 5 buffer (20ml) and brine (20ml), then dried and evaporated to give an oil. The oil was chromatographed on silica using ER as eluent to give a solid (0.58g) which was recrystallised from 2:1 ER-isopentane (15ml) to give the title compound (0.484g), m.p. 86-88°. $[\alpha]_D^{21.5} = -13.66°$ ($CHCl_3$).

Analysis Found: C, 72.5; H, 7.5; N, 2.7.
$C_{29}H_{35}NO_5$ requires: C, 72.9; H, 7.4; N, 2.9%.

Example 4

(±)-6-Hydroxybicyclo[3.2.0]hept-2-en-6-sulphonic acid, compounds with (+)-α-methylbenzenemethanamine (1:1)

(+)-α-Methylbenzenemethanamine (1.12g) was added to an efficiently stirred solution of sodium metabisulphite (1.9g) in water (3 ml) at room temperature. The pH of the resulting mixture was then adjusted to 6-7 by the dropwise addition of glacial acetic acid. (±)-Bicyclo[3.2.0]hept-2-en-6-one (1.08g) was then added and the mixture stirred for 5min. The resulting precipitate was then isolated by filtration, washed with ER (5ml) then dried under reduced pressure to give the title compound as a crystalline solid (2.32g), $[\alpha]_D^{25}$=+1.7° (C=5, water).

Example 5

(-)-6-Hydroxybicyclo[3.2.0]hept-2-en-6-sulphonic acid, compound with (+)-α-methylbenzenemethanamine (1:1).

The title compound of Example 4 (20g) was recrystallised from n-butanol (40 ml) to give a partially resolved product (9.52g), $[\alpha]_D^{25}$= -8.8°. A further recrystallisation of this material from propan-2-ol (28 ml) gave the title compound as a crystalline solid (5.8g), $[\alpha]_D^{25}$= -15° (C=5, water), m.p. 86-88°.

CLAIMS

1. A method of resolving a racemic bicycloheptenone of formula (1)

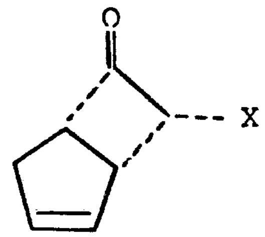

(1)

(in which X is a hydrogen, chlorine or bromine atom) which comprises forming an α-hydroxysulphonic acid - chiral amine salt thereof of formula (2)

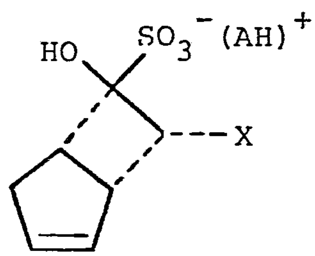

(2)

(in which A is the chiral amine), separating the diastereomeric salts and regenerating the bicycloheptenone of formula (1) in optically active form from the separated salt.

2. A method as claimed in claim 1 in which the chiral amine is R-(+)-1-phenylethylamine.

3. A method as claimed in claim 1 or claim 2 in which X is a hydrogen atom.

4. A method as claimed in any one of the preceding claims in which the salts of formula (2) are separated by fractional crystallisation.

5. Amine salts of the formula (2) as defined in claim 1.

6. Resolved bicycloheptenones of formula (1) as defined in claim 1.

7. (-)-Bicyclo[3.2.0.]hept-2-en-6-one.

8. The compound as claimed in claim 9 characterised by an $[\alpha]_D^{23}$ of about -33.2° (c = 5, $CH_3OH$).

9. Prostanoids and other substituted cyclopentanes when prepared from a resolved bicycloheptenone prepared by a method as claimed in any one of claims 1 to 4.